# EUROPEAN PATENT SPECIFICATION

(11) **EP 4 688 199 B1**
(45) Date of publication and mention of the grant of the patent: **09.12.2026**
(21) Application number: 24717102.8
(22) Date of filing: 26.03.2024
(51) Int. Cl.: B01D 15/18, B01D 15/20, B01J 20/28, C07K 1/14, B01D 15/36, B01D 15/38, B01D 15/32, B01J 20/08, B01J 20/10, B01J 20/24, B01J 20/26, B01J 20/285, B01J 39/05, B01J 39/26, B01J 41/07, B01J 41/20, B01J 47/02, C07K 1/18, C07K 1/34, C07K 1/36, B01J 47/127

(54) **NOVEL MATERIALS AND METHODS FOR REFINING PROCESSES**
NEUE MATERIALIEN UND VERFAHREN ZUR VERFEINERUNG VON VERFAHREN
NOUVEAUX MATÉRIAUX ET MÉTHODES POUR DES PROCÉDÉS DE RAFFINAGE

(30) Priority: 26.03.2023 DK PA202300275
(43) Date of publication of application: 11.02.2026
(73) Proprietor: LPS Biosolutions A/S, 2750 Ballerup (DK)
(72) Inventor: LIHME, Allan Otto Fog, 3520 Farum (DK); SOMMER-KNUDSEN, Jens, 3520 Farum (DK)
(74) Representative: Plougmann Vingtoft a/s
(86) International application number: PCT/DK2024/050074
(87) International publication number: WO 2024/199610

(56) References cited:
- US-A1- 2015 299 248
- US-A1- 2017 298 092
- US-A1- 2022 081 469
- US-B2- 6 866 783

## Description

### Technical field of the invention

The present invention relates to a method and apparatus for isolating one or more target biological molecule(s) from a biological composition. In particular, the present invention relates to isolating one or more target biological molecule(s) from a biological composition by dynamic packed bed adsorption using a separation medium comprising porous rod-shaped particles linking to the target molecule, and filtration using a filter having a pore size of at least 1.5 µm and wherein the filter is a tangential flow filter.

### Background of the invention

There is an increasing need for efficient and cost effective, preparative methods for refining of biological molecules from compositions such as extracts or secretions of plants, animals, fungi, protists, bacteria or archaea, cell supernatants, cell lysates, fermentation broths, dairy streams and blood. Examples of molecules that need to be refined or purified include, but are not limited to, proteins, peptides, polysaccharides, lipids, nucleic acids, small molecules, cells, extracellular vesicles and combinations thereof. A significant number of methods have previously been described and employed for such processes including partitioning, precipitation, solid phase extraction, membrane processes, other techniques, and combinations thereof.

Known methods for refining and purification of biomolecules may for example be by using selective adsorption of molecules from a liquid solution or suspension onto a solid material. These materials are often referred to as separation matrices, and a number of materials have been used for such separations. A typical technique utilising selective adsorption of molecules from a liquid phase onto a solid support is chromatography, and several natural and synthetic materials such as agarose, dextran, carrageenan, cellulose, regenerated cellulose, derivatised celluloses, polyacrylamide, polymethacrylate, polyacrylonitrile, polyethylene oxide, polystyrene, polyamide, polyester, polysulfone, polytetrafluoroethylene, polyvinylidene fluoride, silica, alumina, hydroxyapatite, ceramic materials and various combinations of these and other materials have been used for this purpose. The solid supports are typically in the form of particles, monoliths or membranes. Commonly, the particles used are in the form of spheres that are packed in columns through which the mobile phase containing the target molecules are passed. Packing of these spherical particles must be done carefully, as a phenomenon known as channelling can significantly reduce the efficiency of a packed bed column.

In general, there are two main drawbacks with traditional packed bed columns using porous media such as agarose beads (typically the media used are agarose beads with a diameter between 50 and 300 µm) for large scale separations: backpressure and residence time. This significantly limits the flow rates that can be used, as the flow rate of the mobile phase must not be so high as to induce a collapse of the media causing complete blockage of flow and furthermore the requirement of diffusion of the target molecules into the porous media requires a certain residence time that increases with the diameter of the spherical porous particles. To overcome these limitations other separation media have been developed such as membranes or nanofibres that have been formed into mats (WO2017184706 (A1), US 2017298092 (A1), US11008364 (B2), WO 2013068741(A1), WO 2019137869A1, US 2017298092 (A1)), fabrics (US 5808010 A), non-woven fabrics (US2013122215 (A1), WO 2021/221050), polymeric fibres with entrapped particulate material WO 2004003268 (A1), composite fibres with polymeric and hydrophilic polymers have been grafted onto or otherwise cast around fibrous or fabric backbone (US9295928 (B2), WO 2008107196 A2, Carbajal et al Cellulose Chem. Technol., 54 (1-2), 125-134(2020*)*)) but these media also have a number of limitations. One paper by Zhao et al (R. Zhao et al. / J. Chromatogr. B 816 (2005) 175-181) describes the use of non-porous viscose particles with a length of 100 µm and a diameter of 30 µm, but when these particles were packed in a small column, an extremely high backpressure of 10 MPa was required to obtain a modest flow of 1000cm/h.

US 6 866 783 B2 describes a membrane module for substance-specific treatment of a fluid where the fluid to be treated is to the membrane module in cross-flow mode along the side of the walls of the first membrane elements.

US 2022/081469 A1 discloses a process for purifying biological products in a dynamic filtration modul including tangential flow filtration.

In addition, classical packed bed columns generally suffer from the disadvantage that the biological composition comprising a target biological molecule to be isolated, must be devoid of suspended or colloid particles, such as such as whole cells, cell debris, and aggregates of protein, lipid, and/or polysaccharide, which would otherwise tend to accumulate inside the packed bed separation medium and eventually clog up the system with increased back pressure and loss of flow through the column as the result. Removing such suspended or colloid particles from the biological composition prior to packed bed adsorption in industrial scale applications is a highly demanding task which lead to product and productivity losses. Plant juices, plant tissue extracts, and fermentation based biological compositions may be particularly intractable with respect to the removal of suspended and/or colloid particles in an industrial setting requiring processing of many cubic meters of the composition per hour.

In other adsorption-based separation systems, the particles constituting the separation medium are employed in a suspended state and/or in a dynamic packed bed state. This is, for example, the case in the separation methods and equipment disclosed in Danish patent applications PA 2021 70471 and PA 2022 70361 and international patent application PCT/DK2022/050195. In these systems the separation medium may be retained on filters having a porosity which is close to the size of the separation medium. For example, the filter may have a nominal porosity of 20 micron and the separation medium may have an average diameter of 60 micron. The advantage of such a system is that impurities in the form of suspended and/or colloid particles in the feed material having a size substantially less than 20 micron may freely pass the filters, and thereby the need for removing such particles from the feed prior to adsorption is eliminated. However, when the particles of the separation medium are substantially spherical it is often observed that the filters are partly blocked by the separation medium and therefore frequent back flushing for maintenance of high filtration flow rates is necessary. Also, when the particles of the separation medium have an average diameter close to the pore size of the filter, it is often observed that some of the particles unintendedly pass the filter and are hereby lost. This is especially the case when the particle size diameter has a significant variance from the average diameter of the particles, which is frequently the case for separation media employed in industrial scale adsorption processes. Attempts to solve this issue e.g., by employing spherical particles of larger diameter has been found to significantly decrease the binding capacity of the separation medium and increase the time it takes to achieve binding equilibrium.

It would therefore be advantageous to have a method and apparatus for isolating biological molecule(s) from a biological composition using a particulate separation medium that avoids, or minimizes, blocking of filters and losses of the separation medium employed in systems wherein the separation medium is applied in a suspended or dynamic packed bed state as described above. It would in particular be advantageous to have such a method and apparatus for isolating biological molecule(s) directly from a biological composition comprising suspended and/or colloid particles using a particulate separation medium that avoids, or minimizes, blocking of filters and losses of the separation medium employed in a suspended or dynamic packed bed state allowing said suspended and/or colloid particles to pass freely as described above.

### Summary of the invention

Thus, an object of the present invention relates to a method for isolating one or more target biological molecule(s) from a biological composition using a separation medium that overcomes the above-mentioned problems with inefficient flow, blocking of filters and losses of separation medium. This is obtained with the method and apparatus of the present invention that allows for high flow rates and short residence times.

One of the aspects of this invention is to provide a method for isolating one or more target biological molecule(s) from a biological composition comprising the steps of
a) providing a separation medium in the form of porous rod-shaped particles that has been derivatised with functional ligands to interact with target biological molecule(s) in a mobile liquid phase, and wherein the porous rod-shaped particles have an average length from 50 µm to 10,000 µm and average diameter from 1 µm to 350 µm;
b) mixing the separation medium with a biological composition comprising one or more target biological molecule(s), and whereby the one or more target biological molecule is adsorbed onto the separation medium;
c) filtrating the mixture of separation medium and biological composition using a filter to form a dynamic packed bed of the separation medium on the filter, wherein the pore size of the filter is at least 1.5 µm, and wherein the filter is a tangential flow filter;
d) optionally, eluting the target biological molecule from the separation medium while the separation medium is being retained by said filter.

A second aspect of the invention relates to an apparatus for isolating one or more target biological molecule(s) from a biological composition, the apparatus comprising:
- a first inlet 1 to a mixing tank 2 comprising a separation medium in the form of porous rod-shaped particles that has been derivatised with functional ligands to interact with target biological molecule(s) in a mobile liquid phase, and wherein the porous rod-shaped particles have an average length from 50 µm to 10,000 µm and average diameter from 1 µm to 350 µm;
- said mixing tank 2 having one or more first fluid connections 3 leading fluid from the first inlet 1 to a first end 4 of a tangential flow filtration unit 5 comprising a filter 6 with a pore size being at least 1,5 µm; and wherein the filter is a tangential flow filter;
- and wherein a second end 7 of the tangential flow filtration unit 5 has a second fluid connection 8 leading retentate back to the mixing tank 2 and the first end of the tangential flow filtration unit 5;
- the tangential flow filtration unit 5 having a third fluid connection 9 leading permeate to a first outlet 10.

The separation medium used in the method and apparatus of the present invention comprises porous rod-shaped particles that allows for packing in a dynamic layer, including dynamic and tangential dynamic packing on filters. This packing of a layer of the rod-shaped particles on the filter may also be referred to as a dynamic packed bed. Further, the present invention relates to a method and an apparatus for purifying and isolating biological molecules using a separation medium in the form of porous rod-shaped particles, where the porous rod-shaped particles have an average length from 50 µm to 10,000 µm and an average diameter from 1 µm to 350 µm.

In a particular aspect of the invention the target biological molecule is isolated from a biological composition comprising suspended and/or colloid particles such as whole cells, cell debris, protein, lipid, and/or polysaccharide aggregates.

### Brief description of the figures

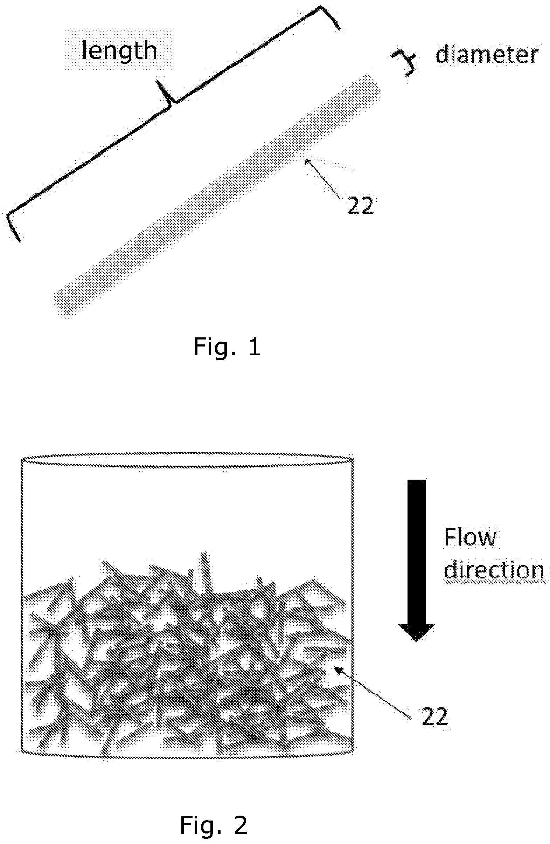

**Figure 1** shows a porous rod-shaped particle (22).
**Figure 2** shows the porous rod-shaped particle (22) packed in a traditional axial column.

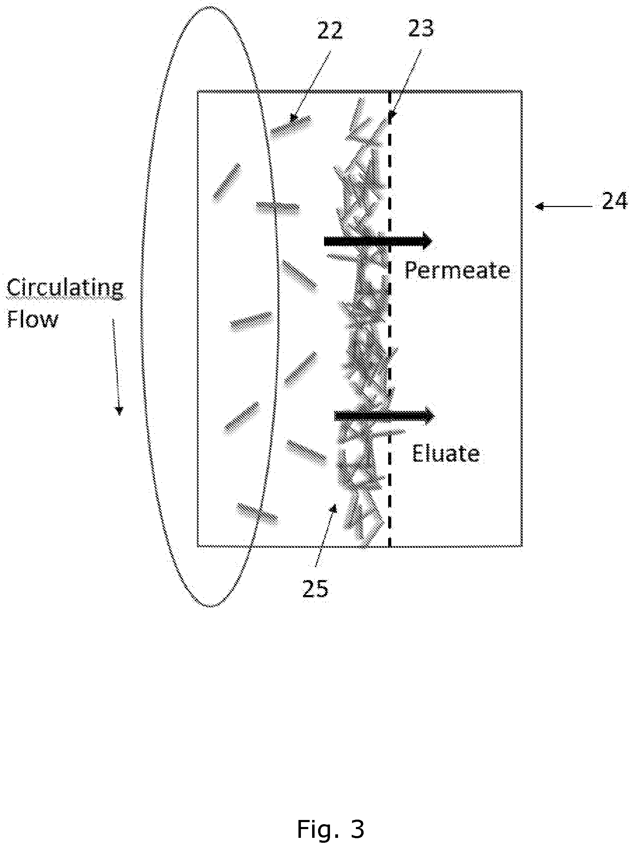

**Figure 3** shows a schematic representation of the circulating fluid flow over the membrane/filter (23) of the tangential flow filtration unit of the separation apparatus where the arrows indicate the flow of permeate and eluate from the retentate side to the permeate side. Furthermore, a dynamic layer (25) of rod-shaped particles (22) is illustrated by means of rod-shaped particles forming the dynamic layer reaching a certain thickness.

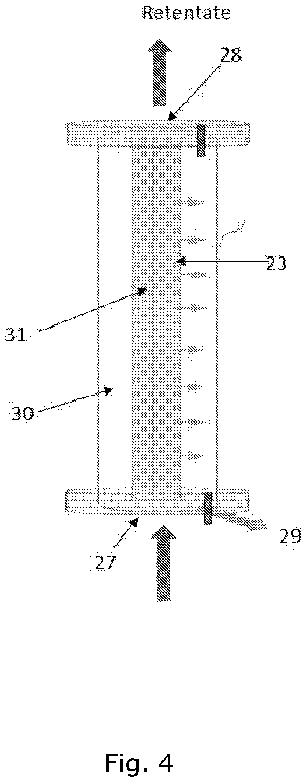

**Figure 4** shows a schematic representation of a tangential flow filtration unit (26) of the separation apparatus having a membrane/filter (23) in the shape of a filter tube, an inlet (27) and an outlet (28) for the retentate as well as a permeate outlet (29). A permeate compartment (30) is formed between the filter house and the filter tube (31). The permeate is filtrated into the permeate compartment (30).

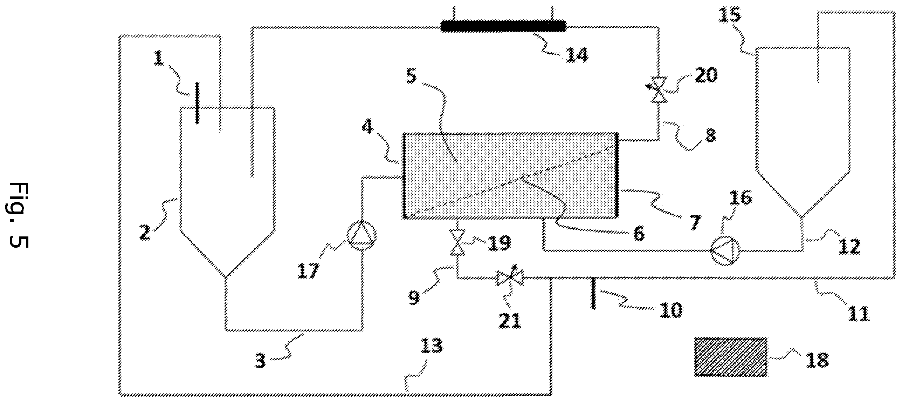

**Figure 5** shows, a schematic representation of an apparatus according to the present invention comprising a first inlet 1 connected to a mixing tank 2 which is further connected to first fluid connection(s) 3 leading fluid to a tangential flow filtration unit. This apparatus is further equipped with a second connection 8 leading retentate back to the mixing tank and a third connection 9 that leads permeate to a first outlet 10.

The present invention will now be described in more detail in the following.

### Detailed description of the invention

### DEFINITIONS

Prior to discussing the present invention in further details, the following terms and conventions will first be defined:

### Dynamic packed bed

In the present context, the term "dynamic packed bed" refers to a separation medium being temporarily packed or concentrated to a porous cake on a filtering surface during a separation cycle, wherein the separation medium is resuspended or otherwise physically rearranged during the separation cycle, e.g., by applying a cross-flow, backflushing, shaking or vibration. The dynamic packed bed may also be referred to as a dynamic layer.

### Separation medium

In the present context the term "separation medium" has the same meaning as the term "sorption material"

### Sorption material

In the present context, the term "sorption material" refers to a material that is able to attach to a specific compound or group of compounds, such as a proteins or other biomolecules. A sorption material may also be named an adsorbent.

### Adsorbent

In the present context, the term "adsorbent" refers to a material, which is able to interact with a specific compound or group of compounds which are thereby reversibly attached to the surface of the material. For example, the adsorbent may comprise ligands able to interact with the specific compound, such as but not limited to: ion exchange ligands, hydrophobic ligands, affinity sorption ligands, and mixed mode ligands. An adsorbent may be substantially impermeable whereby only the outer surface is available for interaction with compounds, or it may be highly porous with pores large enough to allow compounds to diffuse into and interact with the inner surface of the adsorbent. Alternatively, the adsorbent may comprise an immobilized enzyme which is bound by reversible adsorption, physical entrapment or by covalent chemical bonding to the protein sorption material and for enzymatic modification of the compound present in the composition, such as but not limited to controlled hydrolysis of proteins in the composition into peptides by an immobilized protease, or modification of carbohydrates e.g. polysaccharides.

Further, the term "adsorbed" as in the biological molecule is adsorbed onto the separation medium refers to the biological molecule interacts with ligands in the rod-shaped particles of the separation medium. Hereby, the biological molecule is adsorbed to the separation medium.

### Elution liquid

In the present context, the term "elution liquid" refers to a liquid substance or composition, which is capable of releasing a compound attached to a sorption material.

### Circulating flow

In the present context, the term "circulating flow" refers to the flow of the part of the composition, mixture, liquid, fluid, or suspension that enters the tangential flow filtration unit in the first end and leaves it from the second end as retentate that is circulated back to the first end, such as through the mixing tank.

### Tangential flow filtration unit

In the present context, the term "tangential flow filtration unit" or cross-flow filtration unit, refer to filtration equipment wherein the filter surface is tangential to the flow direction of the composition, which is to be filtered.

### Filter

In the present context, the term "filter" refers to a filter or a membrane useful for separating components of a composition.

### Flux

In the present context, the term "flux" or "flux rate" means the volume of liquid passing one square meter of filter area per hour. The unit applied in the art is LMH (i.e. L/m2/h).

### Nominal pore size

In the present context, the term "nominal pore size" relates to the pore size of a filter at which a minimum percentage of components larger than that pore size should be retained by the filter. For example, a nominal pore size of 60% for a filter having an average pore size of X, is considered to retain at least 60% of particles which are larger than X.

### Retentate

In the present context, the term "retentate" relates to a composition which has passed over a filter. Thus, a retentate is the part of a filtered composition which was retained by the filter (i.e. retained on the retentate side of the filter, see figure 3).

### Permeate

In the present context, the term "permeate" relates to a composition which has passed through a filter. Thus, a permeate is the part of a filtered composition which went through the filter (i.e. passed through the filter to the permeate side of the filter, see figure 3).

### Backward flush

In the present context, the term "backward flush" relates to an event wherein the flow of composition through the filter is reversed.

### Pulsed pressure increase and/or backward flush of the permeate

In the present context, the term "pulsed pressure increase and/or backward flush of the permeate" relates to an event wherein the pressure on the permeate side is increased. For example, the pressure increase may be large enough that the difference between the pressure on the retentate side and the pressure on the permeate side of the filter is essentially zero, whereby the flow of composition through the filter may be reversed to give a weak backward flush though the filter. In another example, the pressure increase on the permeate side is much larger than the pressure on the retentate side, whereby a strong backward flush is applied.

### Biological composition

In the present context, the term "biological composition" refers to a complex composition obtained from a biological source, such as plants, microorganisms and animals, and being a liquid, suspension or mixture. For example, the biological composition may be milk, whey, fermentation broth, plant extracts or blood plasma.

### Biomolecules

In the present context, the term "biomolecules" refers to molecules that are produced by cells and living organisms.

### Extractive fermentation

In the present context "extractive fermentation" is a bioprocess that involves the separation of a product from the fermentation broth in situ, while the fermentation is ongoing. This is done to prevent product inhibition, increase product concentration, or minimize the impact of impurities on the downstream purification process. Adsorption involves the use of a separation medium that selectively adsorbs the product, which can then be eluted and recovered. Extractive fermentation can be used for the production of a wide range of products, including organic acids, amino acids, antibiotics, enzymes, monoclonal antibodies and other bio-pharmaceuticas. It offers several advantages over traditional fermentation processes, such as higher product yields, reduced product inhibition, and simplified downstream processing.

The term "absorbance at 600 nm" means an expression of the amount of light of wavelength 600 nm passing through a liquid sample when measured in a spectrophotometer using 10 mm light path cuvettes. The absorbance of a liquid sample is often expressed as O.D. 600 nm.

### Refining

In the present context, the term "refining" refers to a process wherein a protein or other biomolecule is concentrated, separated, cleaned, purified, and/or isolated from a biological composition.

### Separation

In the present context, the term "separated" refers to something which has been set apart from something else. More specifically, it may be a protein which has been set apart from other species of a composition, such that a new composition comprising the protein is obtained.

### Transmembrane pressure (TMP)

In the present context, the term "transmembrane pressure" is defined as:
$TMP = \left(feed pressure+retentate pressure\right) / 2 - permeate pressure .$

### SEPARATION MEDIUM IN THE FORM OF POROUS ROD-SHAPED PARTICLES

Surprisingly, it has been found that a separation medium in the form of porous rod-shaped particles shows a significantly decreased tendency to block the filters employed in separation techniques wherein the medium is employed in a suspended and/or dynamic packed bed state, e.g., as described in Danish patent applications PA 2021 70471 and PA 2022 70361 and international patent application PCT/DK2022/050195. Further, it has surprisingly been found that the separation medium in the form of the porous rod-shaped particles can be withheld by filters even though the pores of the filters are about the same size or larger than the diameter of the porous rod-shaped particles.

In tangential flow separation applications such as those described in Danish patent applications PA 2021 70471, and PA 2022 70361 and international patent application PCT/DK2022/050195, the rod-shaped particles according to the invention provide an excellent solid separation matrix that can easily form a dynamic binding layer allowing for very high flow rates and short residence times. The rods according to the invention have a large surface area compared to spherical media and the randomised packing bed allows for high flow rates and low back pressures while reducing the chance of channelling of the biological compositions through the separation media. As such, they provide an excellent separation media for use in high flow tangential flow based separations.

In the context of the present invention, the porous rod-shaped particles may also be referred to as "rods".

The rods can be made from a number of materials, including, but not limited to, agarose, dextran, carrageenan, cellulose, regenerated cellulose such as viscose, rayon or lyocell, derivatised celluloses, polyacrylamide, polymethacrylate, polyacrylonitrile, polyethylene oxide, polystyrene, polyamide, polyester, polysulfone, polytetrafluoroethylene, polyvinylidene fluoride, polyvinyl alcohol, silica, alumina, hydroxyapatite, ceramic materials and various combinations of these. The rods are derivatised with functional ligands to enable their application in various types of separation technologies, including, but not limited to, cation and anion exchange, mixed mode, hydrophobic interaction, affinity, metal ion affinity, metal chelation and group specific adsorptions. In addition, the rods may be strengthened by chemical cross-linking.

The rods according to the invention have an average length from 50 µm to 10,000 µm and an average diameter from 1 µm to 350 µm. In some embodiments the rods will have an average length from 75µm to 2,000µm, such as from 100µm to 1,500µm, for example 100µm to 1,000 µm, preferably form 200µm to 600µm, while the average diameter is from 5 µm to 180 µm, such as 6 µm to 100 µm, preferably 7 µm to 50 µm. In other embodiments of the invention, the rods will have an average length from 100 µm to 1,000 µm and an average diameter from 5 µm to 180 µm, and in further embodiments the rods will have an average length from 200 µm to 600 µm and an average diameter from 7 µm to 50 µm.

For certain applications directed towards isolation of relatively low molecular weight biomolecules the average diameter of the rods will preferably be in the range of 75µm to 350 µm, such as in the range of 100 µm to 250 µm, such as in the range of 120 µm to 200 µm, such as in the range of 120 µm to 180 µm.

Preferably the variance of the diameter of the rods is at a minimum. In one embodiment at least 80 %, such as at least 90 %, such as at least 95 %, such as at least 98 % of the rods have a diameter within the range of 30 % to 300 % of the average diameter, such as within the range of 40 % to 250 % of the average diameter, such as within the range of 50 % to 200 % of the average diameter, such as within the range of 60 % to 180 % of the average diameter, such as within the range of 70 % to 160 % of the average diameter, such as within the range of 75 % to 125 % of the average diameter.

For example, at least 80% of the rods have a diameter within 30% to 300% of the average diameter. Preferably, at least 90% of the rods have a diameter within 50% to 200% of the average diameter. Even more preferably, at least 90 % of the rods have a diameter within 75 % to 125 % of the average diameter.

In an embodiment of the invention, the ratio between the average diameter and the average length of the rods is at least 1:2, such as at least 1:3, such as at least 1:4, such as at least 1:5, such as at least 1: 6, such as at least 1:7, such as at least 1:8, such as at least 1:10, such as at least 1:12, such as at least 1:14, such as at least 1:16, such as at least 1:18, such as at least 1:20, such as at least 1:25.

In a further embodiment of the invention, the ratio between the average diameter and the average length of the rods is in the range of 1:2 to 1:1000, such as in the range of 1:5 to 1:500, such as in the range of 1:5 to 1:200, such as in the range of 1:5 to 1:100. For example, the ratio between the average diameter and the average length is in the range of 1:5 to 1:50; such as in the range of 1:5 to 1:30, preferably in the range of 1:10 to 1:100, such as in the range of 1:15 to 1:70, most preferably in the range of 1:15 to 1:50.

Preferably, the average diameter of the rods is about the same size or smaller than the diameter of the pores of the filter used to retain the rods. Hence, in an embodiment of the present invention, the pore size of the filter is equal to or larger than the diameter of the rod-shaped particles.

Thus, in a preferred aspect of the invention the average diameter of the rods is within the range of 25-300 %, such as within the range of 35-250 %, such as in the range of 45-225 %, such as in the range of 50-200 %, such as in the range of 60-180 %, such as in the range of 70-160 %, such as in the range of 80-150 % of the average diameter of the pores of the filter used to retain the rods during the adsorption process.

In a further embodiment of the invention the average diameter of the rods is within the range of 25 - 150 %, such as within 35-125 %, such as within 45-100 %, such as within 50-100 %, such as within 60-100 %, such as within 70-100 % of the average diameter of the pores of the filter used to retain the rods during the adsorption process.

The rods used in the method and apparatus of the invention are porous such that large molecules like proteins having a molecular weight higher than 10 kD, such as higher than 50 kD, such as higher than 100 kD are able to migrate into the interior volume of the rods. The pores influence the adsorption of the target compound by the rods as it allows migration and binding of compounds to the material surfaces presented inside the volume of the rods. In one embodiment, at least 90 %, such as 95 %, such as 98 % and preferably at least 99 % of the pore volume of said rods are in the range of 10 nm to 300 nm, such as in the range of 20 nm to 250 nm, such as in the range of 30 nm to 200 nm. The pore diameter is conveniently determined by porosimetry using mercury intrusion.

The term "porous" refers in the context of the present invention to that the rods comprises pores, channels, openings or holes inside the rods, such that for example liquid can pass through the rod.

The rods may have a broad range of cross-sectional shapes, such as a substantially cylindrical cross-sectional shape, a flattened shape, a triangular or elongated triangular shape, a needle shape, a lenticular shape, trilobal, scalloped oval and/or mixtures of these.

To improve the porosity of rods made from cellulose, viscose, rayon, lyocell and similar materials, a mercerisation process was optionally conducted prior to any cross-linking or derivatisation. Such mercerisation was conducted by soaking the rods in an aqueous solution of sodium or potassium hydroxide at concentration of 2-10 M for 1-5 hours.

### METHOD FOR ISOLATING ONE OR MORE TARGET BIOLOGICAL MOLECULE(S) FROM A BIOLOGICAL COMPOSITION

The method of the present invention for isolating one or more biological molecule(s) from a biological composition involves filtration using a filter.

### FILTER

### FILTER PORE SIZE

The tendency of fouling of the filter may be reduced by applying filter types with increased porosity allowing a fraction of the material to pass through the filter into the permeate. Increased pore size also allows for increased flux rate and thus, a faster and more cost efficient process. Furthermore, the inventors of the present invention surprisingly found that by reducing the fouling of the filter, the ability of a backward flush system to apply a suitable backward flush for retaining high flux during operation gets strongly enhanced.

Thus, the nominal pore size of the filter should be as large as possible, but still retain the porous rod-shaped particles (sorption material). An embodiment of the present invention relates to the filter having a nominal pore size of at least 1.5 µm. For example, the filter has a nominal pore size of at least 5µm, such as at least 10 µmm preferably at least 20 µm, more preferably at least 30 µm and more preferably at least 40 µm.

In a further embodiment of the invention, the filter has a nominal pore size in the range of 1.5 µm to 300µm, such as 5 µm to 250 µm, and preferably in the range of 10 µm to 220 µm, more preferably in the range of 20 µm to 200 µm.

In an embodiment of the present invention, the pore size of the filter may be equal to or larger than the diameter of the porous rod-shaped particles.

In the context of the present invention the term "equal to" refers to the filter is having about the same pore size as the diameter of the rods. With the term "equal to" means that the pore size of the filter and the diameter of the rods is having 90% to 100% identity, i.e. the "equal to" means that there can be up to 10% difference. Preferably, the difference of "equal to" is less than 5%, and more preferably less than 2%.

An embodiment of the present invention relates to the apparatus described herein, wherein the filter has a nominal pore size in the range of 1.5 to 300 µm. Yet, another embodiment of the present invention relates to the apparatus described herein, wherein the filter has a nominal pore size in the range of 5 to 250 µm, such as 10 to 200 µm, such as 10 to 100 µm, such as 10 to 50 µm. The filter may also have a nominal pore size of 5 to 50 µm, such as 7 to 40 µm, preferably 10 to 30 µm. However, for some applications wherein the raw material contains larger insoluble particles, including living cells such as bacteria, yeast and mammalian cells, the filter has a large nominal pore size. An embodiment of the present invention therefore relates to the apparatus described herein, wherein the filter has a nominal pore size in the range of 20 to 300 µm, such as 30 to 250 µm, such as 30 to 200 µm, such as 30 to 100 µm, such as 30 to 60 µm, such as 40 to 200 µm, such as 50 to 200 µm.

### FILTER AND FILTER DEVICE

The filter for separation of the rod-shaped particles according to the invention is of a porous inorganic material and/or porous organic polymer material. A specific embodiment of the present invention therefore relates to a filter material selected from the group consisting of sintered metal, sintered metal powder, sintered metal strings and layers hereof, sintered metal oxides, sintered ceramics, porous glass, polypropylene, polyethylene, cellulose, cellulose acetate, viscose, polysulfone (PSU), polyethersulfone (PES), polyarylethersulfone (PAES), polyvinylidinedifluoride (PVDF), polytetraflouorethylene (PTFE), polyamide, nylon, polyester, surface modified PES, surface modified PTFE, and mixtures thereof.

An embodiment of the present invention relates to a filter material for separation of the rod-shaped particles according to the invention comprising porous sintered stainless steel particles or porous sintered stainless steel mesh.

A further embodiment of the present invention relates to a filter for separation of the rod-shaped particles according to the invention comprising a porous organic polymer material selected from the group consisting of polypropylene, polyethylene, cellulose, cellulose acetate, viscose, polysulfone (PSU), polyethersulfone (PES), polyarylethersulfone (PAES), polyvinylidinedifluoride (PVDF), polytetraflouorethylene (PTFE), polyamide, nylon, polyester, surface modified PES, surface modified PTFE, and mixtures thereof.

A further particular embodiment of the present invention relates to a filter for separation of the rod-shaped particles according to the invention comprising a polypropylene filter.

Another particular embodiment of the present invention relates to a filter for separation of the rod-shaped particles according to the invention comprising a polyethylene filter. Yet another particular embodiment of the present invention relates to a filter comprising a polyamide filter, such as a nylon filter. Just another particular embodiment of the present invention relates to a filter comprising a melt blown polypropylene filter.

The filters according to the present invention may have a variety of shapes and forms. Particularly relevant are filters designed for cross-flow filtration wherein the retentate liquid is passing the filtering surface in a direction perpendicular to the direction of the filtrate/permeate flow. Cross-flow filtration devises are often also named tangential flow filtration devices, as opposed to dead-end filtration devices wherein the liquid is passing directly through the filtering surface. Cross-flow filtration devices may be in the form of hollow fibers, tubular or cylindrical, and they may be in the form of flat sheets, discs or plates. Flat sheet cross-flow membranes may be spiral wound or they may be stacked with spacers in between each membrane layer. The cross-flow filters may further be designed for maximum flux and stabilization of flux by vibration, shaking, rotation or spinning. In an embodiment of the present invention the filter unit is designed for applying backward flushes during operation of the filtration process. Also, the filter and the filtration device may be designed for hygienic operations enabling efficient cleaning of the system before and after operation.

An embodiment of the present invention relates to a filter for separation of the rod-shaped particles according to the invention, wherein the is a hollow fibre filter, tubular or cylindrical, flat sheet filter or flat screen filter.

In a particular embodiment of the present invention relating to a filter for separation of the rod-shaped particles according to the invention, the filter is a hollow fibre filter or a tubular or cylindrical filter.

In a particular embodiment of the present invention the filter is a hollow fibre filter, or a tubular or cylindrical filter being vibrated or shaked during operation.

For reasons of their surprisingly lower tendency to get blocked by the adsorbent during operation, and their stability towards back flushing and back pulsing it is particularly preferred that the filter is a hollow fibre or a tubular or cylindrical filter.

When the filter is a tubular hollow fibre filter or cylindrical filter, the inner diameter of the filter should be in the range of 2-1000 mm. An embodiment of the present invention therefore relates to a filter for separation of the rod-shaped particles according to the invention wherein the inner diameter of the filter is in the range of 2-800 mm, such as 3-800 mm, such as 4-800 mm, such as 5-800 mm, such as 10-800 mm, such as 20-800, such as 30-800 mm, such as 40-800 mm, such as 50-800 mm, such as 50-700 mm, such as 50-600 mm, such as 50-500 mm.

In a particular embodiment of the present invention the filter is a flat sheet or flat screen being vibrated or shaked during operation.

### METHOD OF THE INVENTION

An aspect of the present invention relates to a method for isolating one or more target biological molecule(s) from a biological composition comprising the steps of
a) providing a separation medium in the form of porous rod-shaped particles that has been derivatised with functional ligands to interact with target biological molecule(s) in a mobile liquid phase, and wherein the porous rod-shaped particles have an average length from 50 µm to 10,000 µm and average diameter from 1µm to 350µm;
b) mixing the separation medium with the biological composition comprising one or more biological molecule(s), and whereby the one or more target biological molecule is adsorbed onto the separation medium;
c) filtrating the mixture of separation medium and biological composition using a filter to form a dynamic packed bed of the separation medium on the filter, wherein the pore size of the filter is at least 1.5 µm; and wherein the filter is a tangential flow filter;
d) optionally, eluting the target biological molecule from the separation medium while the separation medium is being retained by said filter.

In an embodiment of the present invention, the separation medium with the adsorbed target biological molecules is forming a dynamic packed bed on the filter in a flow perpendicular to the tangential flow in the direction of the filtrate flow.

In a further embodiment of the invention, the separation medium after step c) and before step d) is rinsed to remove unbound material.

In an additional embodiment of the invention, the pore size of the filter is equal to or larger than the diameter than of the rod-shaped particles.

A further embodiment of the present invention relates to a method for isolating one or more target biological molecule(s) from a biological composition comprising the steps of
a. providing a separation medium in the form of porous rod-shaped particles that has been derivatised with functional ligands to interact with target biological molecule(s) in a mobile liquid phase, and wherein the porous rod-shaped particles have an average length from 50 µm to 10,000 µm and average diameter from 1µm to 350µm;
b. mixing the separation medium with the biological composition comprising one or more biological molecule, and whereby the one or more target biological molecule is adsorbed onto the separation medium
c. providing means for tangential flow filtration where the separation medium with the adsorbed biological molecules is packed on a filter in a flow perpendicular to the tangential flow in the direction of the filtrate flow
d. optionally rinsing the separation medium to remove unbound material e. eluting the target biological molecules with a suitable liquid and collecting these molecules on the filtrate or permeate side.

A further embodiment of the present invention relates to a method of the invention wherein the method comprises
- a separation apparatus for mixing the separation medium with the biological composition comprising the one or more target biological molecule(s) in step b), and wherein the separation apparatus is capable of circulating the mixture of the biological composition and the separation medium through a tangential flow filtration unit,
- and wherein step c) comprises filtrating the mixture of biological composition and separation medium to obtain a retentate comprising the separation medium with adsorbed target compound, and a permeate, and recirculating at least part of the retentate on the tangential flow filtration unit, gradually forming a dynamic layer of said separation medium with adsorbed target compound, on the retentate site of the filter during the recirculation of the retentate,
- and in step d) adding an elution liquid to release the target biological molecule from the separation medium, and filtrating the retentate using the tangential flow filtration unit to obtain an eluted retentate comprising the separation medium and a target-containing permeate comprising the target biological molecule,
- and optionally, collecting the target-containing permeate and/or the permeate as separated product composition(s).

Hence, the present invention relates to a method for isolating one or more target biological molecule(s) from a biological composition comprising the steps of
a. providing a separation medium in the form of porous rod-shaped particles that has been derivatised with functional ligands to interact with target biological molecule(s) in a mobile liquid phase, and wherein the porous rod-shaped particles have an average length from 50 µm to 10,000 µm and average diameter from 1µm to 350µm;
b. providing a separation apparatus where the separation medium is mixed with a biological composition whereby the target biological molecule is adsorbed by the separation medium, and where the apparatus is capable of circulating the mixture of the composition and the separation medium through a tangential flow filtration unit,
c. filtrating the composition or the mixture to obtain a retentate comprising the separation medium, optionally with adsorbed target compound, and a permeate,
d. recirculating at least part of the retentate on the tangential flow filtration unit, gradually forming a dynamic layer of said separation medium, optionally with adsorbed target compound, on the retentate site of the filter during the recirculation of the retentate,
e. adding an elution liquid to release the target compound from the separation medium, and filtrating the retentate using the tangential flow filtration unit to obtain an eluted retentate comprising the separation medium and a target-containing permeate comprising the target compound
f. optionally, collecting the target-containing permeate and/or the permeate as separated product composition(s).

Still a further embodiment of the present invention relates to a process for separation of a protein or other biomolecule comprised in a biological composition, the process comprising the steps of:
i. mixing a biological composition comprising a target protein or other biomolecule with a composition comprising a sorption material for protein or other biomolecule to obtain a mixture, wherein the target protein or other biomolecule is carried by the sorption material,
ii. filtrating the mixture using a tangential flow filtration unit having a filter with nominal pore sizes in the range of 1.5 to 300 µm for obtaining a first retentate comprising the target protein or other biomolecule and a first permeate which is optionally collected in a waste tank or a permeate collection tank,
iii. recirculating and filtrating the first retentate by returning the obtained first retentate to the tangential flow filtration unit and thereby obtain a second permeate which is optionally collected, and obtaining a second retentate, and wherein the recirculation and further filtration in step iii) is carried out one or more times,
iv. adding an elution liquid to the second retentate to release the target protein or other biomolecule from the sorption material,
v. filtrating the second retentate using the tangential flow filtration unit to obtain a third retentate comprising the sorption material and a third permeate comprising the target protein or other biomolecule;
vi. collecting the third permeate as a separated product composition comprising protein or other biomolecule,
wherein the sorption material is a separation medium in the form of porous rod-shaped particles that has been derivatised with functional ligands to interact with target biological molecule(s) in a mobile liquid phase, and wherein the porous rod-shaped particles have an average length from 50 µm to 10,000 µm and average diameter from 1µm to 350µm.

In a particular embodiment of the invention the biological composition is a live cell culture such as a bacterial or yeast fermentate or a mammalian cell culture.

In a particular embodiment of the invention the separation medium according to the invention is mixed with the live cell culture during or at the end of fermentation in an extractive fermentation process.

In a particular embodiment of the invention the separation medium according to the invention is sterilized and mixed with the live cell culture during fermentation in an extractive fermentation process, wherein a fraction of the separation medium is continuously separated from the fermentation tank by passing a filter allowing passage and return of the living cells to the fermentation tank, while the adsorbent is retained on the filter and optionally washed and eluted to isolate the target protein or other biomolecule.

### THE BIOLOGICAL COMPOSITION AND THE PROTEINS OR OTHER BIOMOLECULES

The separation medium according to the invention may be used for isolation of proteins and biomolecules from a large scope of biological compositions and/or concentration and purification of proteins typically comprised therein. An embodiment of the present invention relates to rod-shaped particles according to the invention able to adsorb one or more proteins or other biomolecule of a biological composition selected from the group consisting of mammalian milk or whey, such as bovine milk, sheep milk, goat milk or camel milk, whey, bovine blood plasma, porcine blood plasma, human blood plasma, egg white, egg yolk, fermentation broth, fermentation broth of genetically modified microorganisms, fermentation broth of mammalian cell cultures, animal tissue extract, plant tissue extract, algae extract, potato tuber extract, and legume extract.

A further advanced embodiment of the present invention relates to the rod-shaped particles as described herein, wherein the sorption material is able to adsorb one or more proteins or other biomolecules of a biological composition, the biological composition being:
- bovine milk, sheep milk, goat milk, or camel milk or whey or a derivative thereof comprising at least one of said proteins selected from the group consisting of lactoferrin, immunoglobulin G, alpha-lactalbumin, and beta-lactalbumin, or
- bovine, porcine, or human blood plasma or a derivative hereof comprising at least one of said proteins selected from the group consisting of albumin, transferrin, immunoglobulin G, immunoglobulin A, alpha-1-antitrypsin, and Factor VIII, or
- egg white or egg yolk or a derivative or mixture hereof comprising at least one of said proteins selected from the group consisting of ovalbumin, lysozyme, immunoglobulin Y, ovomucoid, ovotransferrin, and avidin, or
- plant tissue, potato or legume extract comprising at least one of said proteins or other biomolecules selected from the group consisting of patatin, protease inhibitor, legumin, vicilin, peptides, flavonoids, polyphenols, carbohydrates, polysaccharides, vitamins, phytosterols and glycoalkaloids, or
- fermentation broth of genetically modified microorganisms or a derivative thereof comprising at least one of said proteins selected from the group consisting of cheese rennet, such as bovine chymosin, camel chymosin; enzymes such as amylases, lipases, pollunases, pectinases, xylases, laccases, transglutaminases, phytases, proteases, oxidoreductases, cellulase, chitinase; functional proteins for gelling, emulsification or foaming in food/feed formulations such as rubisco, patatin, legumin, vicilin, protease inhibitor, beta-lactoglobulin, alfa-lactalbumin, ovalbumin, albumin; proteins or peptides having antimicrobial activity such as lactoferrin, transferrin, ovotransferrin, lactoperoxidase, lysozyme, avidin, nicin; sweet tasting proteins or peptides such as thaumatin, brazzein, curculin, mabinlin, monellin, pentadin, anti-freeze proteins including but not limited to antifreeze proteins from plants, fish or other marine animals, and any modified and/or engineered forms of such proteins.

In a further embodiment of the invention, the target biological molecule is isolated from a biological composition comprising suspended and/or colloid particles such as whole cells, cell debris, and aggregated protein, lipid, and/or polysaccharide.

The content of suspended and/or colloid particles in a liquid is often estimated by measuring the amount of light of wavelength 600 nm passing through a liquid sample when measured in a spectrophotometer using 10 mm light path cuvettes. The absorbance of a liquid sample is often expressed as O.D. 600 nm.

In an aspect of the invention the O.D. 600 nm of the biological composition is at least 0.1, such as at least 0.5, such as at least 1.0, such as at least 2.0, such as at least 5.0, such as at least 10,0.

In a particular embodiment of the invention the biological composition is a fermentation broth, and the separation medium is used for isolation of a target protein or other biomolecule in a so-called extractive fermentation process. Extractive fermentation is an approach in biotechnology that combines the processes of fermentation and product separation into a single step where it enhances efficiency and cost-effectiveness. The main advantage of extractive fermentation is its ability to continuously remove products from the fermentation broth, which can improve product yields, reduce the risk of product degradation or inhibition, and lower downstream processing costs.

### THE APPARATUS

In an aspect, the present invention also relates to an apparatus for isolating one or more target biological molecule(s) from a biological composition, the apparatus comprising:
- a first inlet 1 to a mixing tank 2 comprising a separation medium in the form of porous rod-shaped particles 22 that has been derivatised with functional ligands to interact with target biological molecule(s) in a mobile liquid phase, and wherein the porous rod-shaped particles have an average length from 50 µm to 10,000 µm and average diameter from 1µm to 350µm;
- said mixing tank 2 having one or more first fluid connections 3 leading fluid from the first inlet 1 to a first end 4 of a tangential flow filtration unit 5 comprising a filter 6 with a pore size being at least 1,5 µm;
- and wherein a second end 7 of the tangential flow filtration unit 5 has a second fluid connection 8 leading retentate back to the mixing tank 2 and the first end of the tangential flow filtration unit 5;
- the tangential flow filtration unit 5 having a third fluid connection 9 leading permeate to a first outlet 10.

In an embodiment of the invention, the filter in the apparatus has a pore size equal to or larger than the diameter of the rod-shaped particles.

The apparatus of the invention is shown in figure 5. In figure 5, the reference numbers are as follows:
1: inlet
2: mixing tank
3: first fluid connection
4: first end of a tangential flow filtration unit
5: tangential flow filtration
6: filter
7: second end of the tangential flow filtration unit
8: second fluid connection
9: third fluid connection
10: first outlet
11: fourth fluid connection
12: fifth fluid connection
13: sixth fluid connection
14: Heat exchanger.
15: Permeate collection tank.
16: Centrifugal pump.
17: Peristaltic pump.
18: Electronic control units.
19: Opening-closing valve.
20: First variable flow control valve.
21: Second variable flow control valve.

In embodiments of the invention, the filter may be as disclosed earlier under the description of the method and the porous rod-shaped particles may also be as earlier disclosed.

In an embodiment of the invention, the apparatus may also comprise an automatic backward flush system.

It should be noted that embodiments and features described in the context of one of the aspects of the present invention also apply to the other aspects of the invention.

The invention will now be described in further details in the following non-limiting examples.

### Examples

### Materials and Methods:

Maximum Flow rate: Maximum flow rate was measured by packing the rods to a bed height of 15cm in a column with a diameter of 16mm. Flow rate through the column was then increased in increments of 5mL/min until the backpressure in the column reached 1MPa or a maximum flow rate of 100mL/min (3000 cm/h) was achieved.

### Dynamic Binding Capacity (DBC):

Dynamic binding capacity was conducted in a Tricorn 5/50 column with a bed height of 50mm, 3 min residence time at a flow rate of 0.33 mL/min. The DBC is recorded at a 5% break-through of protein.

### Static Binding Capacity (SBC)

Static binding capacity was determined by incubating the rods with excess amount of protein (i.e. at least 2 times the eventual determined capacity) for 12-24 hours, followed by rinsing and elution.

### Example 1

### Mercerisation

### Example 1a

200g dry viscose (rayon) rods with a length of 500 µm and a diameter of 20 µm were suspended in 4000mL of deionised water to which was added 1053 mL of 19M Sodium Hydroxide and left with overhead stirring for 2 hours. After thorough rinsing, the final weight of the swollen, wet rods was 1650g.

### Example 1b

100g dry viscose rods with a length of 500 µm and a diameter of 12 µm were suspended in 2000mL of deionised water to which was added 526 mL of 19M Sodium Hydroxide and left with overhead stirring for 2 hours. After thorough rinsing, the final weight of the swollen, wet rods was 1020g.

### Example 2

### Derivatisation of Rayon rods with ligands containing quaternary ammonium groups

150g wetted and mercerised Rayon rods from Example 1a were mixed with the following under constant stirring:, 88mL water, 21mL 19 M Sodium Hydroxide, 2.5mL of 10M Sodium hydroxide with 12% sodium Borohydride 90g Sodium Sulfate and 38mL 3-Chloro-2-Hydroxypropyl Trimethyl Ammonium Chloride
Maximum flow rate achieved: 50mL/min, equal to 1500cm/h
DBC: 82mg BSA/mL, SBC: 91 mg BSA/mL

### Example 3

### Derivatisation with propane sultone and cross-linking

200g of mercerised Rayon rods from example 1a were mixed with the following under constant stirring84mL deionised water, 17.9mL Sodium hydroxide, 3.33mL of 10M Sodium hydroxide with 12% sodium Borohydride, 66g Sodium Sulfate and : 35.1g Propane Sultone. Mixture was left to react for 16 hours. Following thorough rinsing the total weight of wet fibres was 480g.
Maximum Flow rate achieved: 5mL/min, equal to 150cm/h
DBC: 25mg IgG/mL

### The propane sultone rods were then cross-linked with Epichlorohydrin:

480 g of wet mercerised and propane sultone derivatised rods were then mixed with the following under constant stirring: 380mL demineralised water, 71mL 19M Sodium hydroxide, 1.2g sodium borohydride, 280g sodium sulfate and 24 mL Epichlorohydrin. This mixture was left to react for 16 hours. After rinsing the total weight of wet rods was 480g.
Maximum flow rate achieved: 20mL/min equal to 600 cm/h
DBC: 18 mg/mL SBC: 75 mg/mL

### Example 4

### Derivatisation with 3-Chloro-2-Hydroxypropyl Trimethyl Ammonium Chloride

200g wetted and mercerised Rayon rods from Example 1b were mixed with the following under constant stirring: 118mL water, 28.4 mL 19 M Sodium Hydroxide, 3.33mL of 10M Sodium hydroxide with 12% sodium Borohydride 120g Sodium Sulfate and 50mL 3-Chloro-2-Hydroxypropyl Trimethyl Ammonium Chloride
Maximum flow rate achieved: 10mL/min, equal to 300cm/h
DBC: >91mg BSA/mL, SBC: 115 mg BSA/MI

### Example 5

### Test of Rayon rods prepared as in example 2 in a tangential flow separation system.

Cross-linked Rayon rods prepared according to example 2 were employed in a test run performed in a similar manner to example 1 of Danish Patent application PCT/DK2022/050195. Accordingly, Figure 5 shows a schematic representation of the apparatus used for the example. The mixing tank 2 was a cylindrical stainless-steel tank with a conical bottom and the first fluid connection 3 comprised a variable flow peristaltic tube pump 17 having a maximum flow capacity of 75 L/min. The tangential flow filtration unit 5 was a cylindrical unit equipped with a filter tube made of melt blown polypropylene and having a nominal pore size of 30 micron. The filter tube had a length of 1200 mm, an outer diameter of 30 mm and an inner diameter of 20 mm. The total inner surface filter area of the filter tube was 0.076 m2.

The permeate third fluid connection 9 was connected to a first outlet 10 that would either direct the permeate to a waste tank for collection or through a fourth fluid connection 11 for collection in a permeate collection tank 15. The permeate third fluid connection 9 was further equipped with an opening-closing valve 19 and an electronic control unit 18 (back-pulse controller) to switch the valve position with a chosen frequency. The same controller 18 was controlling a centrifugal pump 16 connected to a permeate back flush port on the tangential flow filtration unit 5 through a fifth fluid connection 12, which pump 16, when activated, reverses the flow of permeate fluid to obtain a pulsed pressure increase/backward flush through the filter tubes. Thus, when the pump 16 was activated, the opening-closing valve 19 at the third fluid connection 9 were closed and liquid delivered by the centrifugal pump 16 then entered the permeate side of the filter unit through the fifth fluid connection 12. The centrifugal pump 16 was connected to the permeate tank 15 and the pulsed pressure increase/backward flush was performed with permeate collected prior to applying the pulsed pressure increase/backward flush.

The apparatus was further equipped with monitors to monitor flow rates, temperature and pressure in all fluid connections. Variable flow control valves 20,21 positioned in the second and third fluid connections 8,9 were used to regulate the flow and thereby also the back pressure drop across the length of the tangential filtration unit 5 and across the filter wall (from retentate to permeate side) in combination with regulation of the flow rate of the variable peristaltic pump 17.

The permeate outlet was directed by a valve such that all permeate was returned to the mixing tank 2 through a sixth fluid connection 13, whereby the system volume remained constant throughout the test.

For testing of the apparatus with water and the rod-shaped separation medium the mixing tank was filled with 20 L of an aqueous suspension of rayon rods prepared as described in example 2. The concentration of the rods was 33 %, as determined by centrifugation of a sample of the suspension for 5 minutes at 3000 G (i.e., a 100 ml sample would have 33 ml sedimented rods after centrifugation). The system volume at the outset of the test was thus 20 L.

### Testing flux rate with and without backward flush

For this test the peristaltic pump was set to a flow rate of 20 L/min whereby the adsorbent suspension was recirculated through the first fluid connection to pass the tangential flow filtration unit and returning to the mixing tank through the second fluid connection. At the outset of the test, the permeate third and fifth fluid connections were fully closed such that no permeate could pass the filtration unit. Under these conditions, the pressure in the first fluid connection measured at the first end of the tangential flow filtration unit was 0.13 bar and the pressure of the second fluid connection measured at the second end of the filtration unit was 0 bar. The pressure gradient between the retentate side of the filters and the permeate side of the filters (TMP) was defined as follows:$TMP = \left(feed pressure+retentate pressure\right) / 2 - permeate pressure$

By opening the second variable flow control valve and applying back pressure by adjusting the first variable flow control valve on the return second fluid connection, TMP was adjusted to set values. For this test, TMP was set to 0.25 bar.

Once the second variable flow control valve was opened and TMP was set to the desired value, a pulsed pressure increase and/or backward flush (BP) i.e., back pressure cycle was started where the opening-closing valve was closed and the centrifugal pump was activated to reverse permeate flow through the fifth fluid connection. The pump was activated for 3 seconds and then the pump was stopped and the opening-closing valve opened again. This cycle was repeated once every minute and the permeate flow was measured 10s after every BP. Once the system had run for 11 minutes, the BP cycle was shut off and any decline of flux over time was observed.

The result of the test indicated that the flux remained practically constant for 10 minutes after shutting of the BP cycle and the small decline in flux observed in this period of time could be completely recovered by applying a single BP cycle for each 10-minute interval of the test.

Conclusion: The use of a separation medium in the form of Rayon rods as prepared in example 2 significantly reduce the need for back flushing compared to the use of spherical particles as described in example 1 of Danish patent application PCT/DK2022/050195, which showed an immediate decrease of flux when the BP cycles were stopped. The test further demonstrated that the back flush system was still effective at ensuring a constant high flux through the filter even at a much lower frequency of the BP.

### Example 6

Example 6 discloses an analysis of using porous rod-shaped particles as adsorption material as compared to using spherical adsorption material.

### Example 6.1 - Derivatization of cellulose fibers without prior maceration.

This example relates to the preparation of derivatized cellulose fibers as rod-shaped particles.

### Cross-linking with epichlorohydrin:

300 g precision cut viscose, V(3.3 Dtex) having a dry diameter of 15.3-18.7 µm (>95%) and a length of 250 µm (Goonvenan Fibers Ltd, UK) was mixed with 6.0 L 0.3 M sodium sulfate and 2.8 L 27.7 % sodium hydroxide under thorough overhead stirring. The temperature was 15 0C.

After 30 minutes stirring, the suspension was added 600 ml epichlorohydrin, and stirring was hereafter continued at 15 0C for 12 hours.

The cross-linked fiber suspension was then separated and washed with 10 L water by vacuum suction on a sintered glass filter. After washing the wet fiber cake was drained for interstitial water. The resulting wet, but drained, fiber cake had a weight of 2700 g and a dry matter content of 10.0 %.

### Preparation of a strong anion exchanger:

500 g of the wet, but drained cross-linked fiber cake was mixed with 1.0 L demineralized water under thorough overhead stirring and added 36 g solid sodium hydroxide and 420 g 2,3-epoxypropyl trimethyl ammonium chloride. The temperature was then raised to 45 0C and reaction under stirring was performed for 12 hours.

The quaternized and cross-linked fiber suspension was then separated and washed with 8 L water by vacuum suction on a sintered glass filter. After washing the wet fiber cake was drained for interstitial water. The resulting wet, but drained, fiber cake had a weight of 787 g and a dry matter content of 8.4 %. When suspended in water the diameter of the fibers was in the range of 45-70 µm (> 90 %) and the length of the fibers was in the range of 260-330 µm (>90 %)

### Preparation of a strong cation exchanger:

500 g of the wet, but drained cross-linked fiber cake was mixed with 1.0 L demineralized water under thorough overhead stirring and added 240 g solid sodium hydroxide. After dissolution of the sodium hydroxide, 100 ml 1,4 butane sultone was added, and the temperature was raised to 70 0C. The suspension was stirred at this temperature for one hour and then further 100 ml 1,4 butane sultone was added. In total 4 x 100 ml 1,4 butane sultone was added with 1-hour intervals under continuous stirring.

The strong cation exchanger fiber suspension was then separated and washed with 12 L water by vacuum suction on a sintered glass filter. After washing the wet fiber cake was drained for interstitial water. The resulting wet, but drained, fiber cake had a weight of 1320 g and a dry matter content of 13.2 %. When suspended in water the diameter of the fibers was in the range of 50-75 µm (> 90 %) and the length of the fibers was in the range of 250-320 µm (>90 %).

### Example 6.2 Derivatization of cellulose fibers 1.4 mm length

### Cross-linking with butanediol diglycidyl ether:

2000 g precision cut viscose, V(3.3 Dtex) having a dry diameter of 15.1-18.9 µm (>95%) and a length of 1.4 mm (Goonvenan Fibers Ltd, UK) was mixed with 20 L water under thorough overhead stirring. Following stirring for 15 minutes 480 g sodium hydroxide was added and the temperature was raised to 35 0C. Then 2840 g anhydrous sodium sulfate was added, and the suspension was stirred for another 15 minutes whereupon 200 ml 1.4 butanediol diglycidyl ether was added. Stirring was hereafter continued at 35 0C for 12 hours.

The cross-linked fiber suspension was then separated and washed with 50 L water by vacuum suction on a sintered glass filter. After washing the wet fiber cake was drained for interstitial water. The resulting wet, but drained, fiber cake had a weight of 7.4 kg and a dry matter content of 23.3 %. The diameter of the fibers in water was in the range of 35-60 µm (> 90 %) and the length of the fibers was in the range of 1.4-2.1 mm (>90 %).

### Example 6.3 Large scale preparation of thin fiber cation exchanger.

### Cross-linking with epichlorohydrin:

6000 g precision cut viscose, V(0.95 Dtex) having a dry diameter of 8.1-9.9 µm (>95%) and a length of 250 µm (Goonvenan Fibers Ltd, UK) was mixed with 80 L 0.3 M sodium sulfate and added 50 % sodium hydroxide to a final concentration of 3.4 M under thorough stirring in a double-walled stainless steel tank. The temperature was 6 0C. After 30 minutes stirring the thick suspension was added 8000 ml epichlorohydrin and stirring was hereafter continued under heating to reach a temperature of 35 0C. Stirring was continued for 12 hours.

The cross-linked fiber suspension was then separated and washed with 500 L water on a vibrating screen filter. After washing the wet fiber cake was drained for interstitial water.

The wet cross-linked fiber cake was then mixed with 80 L 3.4 M sodium hydroxide in the double-walled stainless-steel tank. 8.0 L 1,4 butane sultone was added and the suspension was heated to 70 0C under thorough stirring. The suspension was stirred at this temperature for one hour and then further 8.0 L 1,4 butane sultone was added. In total 4 x 8.0 L 1,4 butane sultone was added with 1-hour intervals under continuous stirring while the temperature was kept constant at approx. 70 0C. Following the last addition of 1.4 butane sultone the suspension was stirred for further 2 hours.

The strong cation exchange fiber suspension was then separated and washed with 750 L water on a vibrating screen filter. During washing with water, the fiber cake was swelling significantly by the uptake of further water. After washing the wet fiber cake was drained for interstitial water. The resulting wet, but drained, fiber cake had a weight of 98.7 kg and a dry matter content of 11.1 %.

When suspended in water the diameter of the fibers was in the range of 30-45 µm (> 90 %) and the length of the fibers was in the range of 250-300 µm (>90 %). The content of sulfonic acid groups was determined by acid-base titration to be 283 micromoles per gram of wet, but drained fibers.

### Example 6.4 Testing permeation of different particles on a mesh filter.

This example demonstrates to which degree different particles pass an 80-µm mesh filter in a static or dynamic mode of operation.

### Particles/fibers tested:

6 % B Agarose Beads Fine (ABT Agarose Bead Technologies, Spain) having a diameter in water in the range of 50 - 70 µm (> 80 %)

Strong cation exchange fibers prepared as disclosed in example 6.1 and with a diameter of the fibers in water in the range of 50-75 µm (> 90 %) and a length of the fibers in the range of 300-360 µm (>90 %).

Butane diol diglycidyl ether cross-linked fibers prepared as disclosed in example 6.2 and with a diameter of the fibers in water in the range of 35-60 µm (> 90 %) and a length of the fibers in the range of 1.4-2.1 mm (>90 %).

### Procedure:

The particles/fibers for testing were drained by vacuum suction on a sintered glass filter. 50 grams of each wet, but drained, particle or fiber preparation was suspended in 5 liters of demineralized water and mixed well.

This suspension was then poured slowly over a 50 x 50 cm sheet of 80 µm filter net (SEFAR NITEX 03-80/31, SEFAR AG, Switzerland) mounted on a 30 cm diameter shaker screen with a bottom stainless steel support plate having 3 mm holes. The filtrate from the shaker screen was collected and subsequently passed over a sheet of 50 x 50 cm 20 µm filter net (SEFAR NITEX 03-20/31, SEFAR AG, Switzerland) without shaking. Thus, any particles/fibers which passed the 80 µm filter net was collected on the 20 µm filter net. The particles/fibers were hereafter collected, drained on a sintered glass filter, and weighed. The relative amount of particles/fibers having passed the 80 µm filter net was calculated as the weight of particles/fibers collected on the 20 µm net/50 x 100 %.

The experiment was first performed with the shaker screen disengaged (i.e. no vibration of the filter net) and then the experiment was repeated with the shaker screen engaged. The result is shown in table 1 below:

**Table 1:**

| **Particle/fiber type** | **Amount passing the 80 µm filter without vibration (%)** | **Amount passing the 80 µm filter with vibration (%)** |
|---|---|---|
| 6 % B Agarose Beads | < 5 % | > 95 % |
| Diameter: 50 - 70 µm | | |
| Fibers | | |
| Diameter: 50-75 µm | < 1 % | < 5% |
| Length: 300-360 µm | | |
| Fibers | | |
| Diameter: 35-60 µm | < 1 % | < 1 % |
| Length: 1.4-2.1 mm | | |

Table 1 shows that spherical beads are largely retained on an 80 µm mesh filter even though the diameter of the beads is significantly smaller than the openings of the filter net when the filter net is static (i.e. not vibrating), while near complete permeation/passing of the filter is happening when the filter is dynamic (vibrating).

Table 1 further shows that fibers of approximately the same diameter as the spherical beads are retained on the 80 µm net irrespective of whether the net is static or dynamic, and the longer fiber of 1.4 mm length is retained most effectively. The consequence of this is that a fiber having substantially the same surface area, and therefore substantially the same mass transport kinetics, as a spherical bead of a given diameter can be applied for dynamic bed adsorption applications using separation filters that have the same or even larger openings than the diameter of the fiber. On the other hand, this cannot be realized with a spherical bead, since they will pass the filter and thus escape the adsorption system.

**Example 7 Isolation of protein from a simulated fermentation broth in cross-flow mode by employing a filter with a pore size substantially equal to the diameter of the fibers.**

Strong cation exchange fibers prepared according to example 6.3 were employed in a test run performed in a tangential flow apparatus as described in example6.1I. Accordingly, Figure 5 shows a schematic representation of the apparatus used for the example. The mixing tank 2 was a cylindrical stainless-steel tank with a conical bottom and the first fluid connection 3 comprised a variable flow peristaltic tube pump 17 having a maximum flow capacity of 75 L/min. The tangential flow filtration unit 5 was a cylindrical unit equipped with a filter tube made of stainless-steel mesh and having a nominal pore size of 40 micron. The filter tube had a length of 600 mm, an outer diameter of 150 mm and an inner diameter of 147 mm. The total inner surface filter area of the filter tube was 0.277 m2.

A simulated fermentation broth containing a secreted target protein was prepared by thoroughly suspending 2.5 kg fresh baker's yeast in 20 L of 50 mM sodium acetate pH 4.7 followed by addition of 2.0 L of a solution of bovine serum albumin (BSA), (Sigma Aldrich, USA, Cat. No.: A2153) at 20 g BSA/L in 50 mM sodium acetate pH 4.7.

Procedure for separation of BSA from simulated fermentation broth:
1) The simulated fermentation broth was poured into the mixing tank. The peristaltic pump was set to a flow rate of 20 L/min whereby the suspension was recirculated through the first fluid connection to pass the tangential flow filtration unit and returning to the mixing tank through the second fluid connection. At the outset of the test, the permeate third and fifth fluid connections were fully closed such that no permeate could pass the filtration unit.
2) After recirculation of the suspension for 5 minutes a 10 ml sample ("mixing tank sample 1") was withdrawn for later analysis.
3) Following this, the valves were opened to allow permeate passing the filtration unit and being recirculated to the mixing tank.
4) A 10 ml sample of the permeate ("permeate sample 1") was collected from the permeate (before going back to the mixing tank) after further 5 minutes recirculation.
5) Then, 2.0 kg of wet, but drained strong cation exchange fibers prepared according to example 6.3 (diameter 30-45 micron) was added in small aliquots to the mixing tank under thorough mixing and constant recirculation and with the permeate passing the filtration unit and returning to the mixing tank.
6) A second, third and fourth mixing tank sample each of 10 ml was collected 1 min, 2, min and 15 minutes after the addition of the strong cation exchanges fibers.
7) After 15 minutes the permeate was now passed to the permeate collection tank and the mixing tank was added 50 mM sodium acetate pH 4.7 with the same flow rate as the permeate flow rate. In this way the mixing tank volume remained constant while the yeast cells and any non-bound BSA was washed out of the apparatus, with the strong cation exchanger being retained by the filter unit.
8) Upon thorough washing with the acetate buffer (until OD280 nm of the permeate was below 0.1) the permeate was again recirculated to the mixing tank and addition of acetate buffer to the mixing tank was halted.
9) To release BSA bound to the strong cation exchanger 2. L of 2 M potassium phosphate buffer pH 7.5 was added to the mixing tank and pH of the suspension in the mixing tank was subsequently adjusted to pH 7.5 with 1 M sodium hydroxide.
10) Following 10 minutes of recirculation the permeate was now again passed to a second permeate collection tank and the mixing tank was added 50 mM potassium phosphate pH 7.5 with the same flow rate as the permeate flow rate. In this way the mixing tank volume remained constant while the released BSA was washed out of the apparatus and collected in the second permeate tank, with the strong cation exchange fibers still being retained within the mixing tank by the filter unit.
11) Upon thorough washing with the phosphate buffer (until OD280 nm of the permeate was below 0.1) the permeate was again recirculated to the mixing tank and addition of phosphate buffer to the mixing tank was halted. A 10 ml sample was then withdrawn from the second permeate tank. ("permeate tank sample 2"). A 10 ml sample was further withdrawn from the mixing tank (mixing tank sample 5)

Mixing tank sample 1 and 2 and Permeate sample 1 were analyzed for the concentration of yeast cells by centrifugation at 4000 G for 10 min in 1.5 ml Eppendorf test tubes and determining the relative volume of yeast cell sediment. The same samples were further analyzed to determine the BSA concentration (by single radial immunodiffusion). The total amount of BSA isolated and recovered in the second permeate tank was determined by analyzing the BSA concentration in permeate sample 2 by single radial immunodiffusion and multiplying with the total volume of permeate collected in the second permeate tank. Mixing tank sample 5 was examined by microscopy to determine whether any yeast cells were still present in the tank.

The yeast concentration and BSA concentration in the samples are shown in table 2 below.

**Table 2:**

| Sample | Yeast concentration (vol/vol %) | BSA concentration mg/ml |
|---|---|---|
| Mixing tank sample 1 | 10 | 1.7 |
| Permeate sample 1 | 10 | 1.7 |
| Mixing tank sample 2 | ND | 0.5 |
| Mixing tank sample 3 | ND | 0.2 |
| Mixing tank sample 4 | ND | 0.05 |

The total amount of BSA isolated and recovered in the second permeate tank was found to be 37.5 g BSA corresponding to approx. 94 % of the BSA added to the simulated fermentation broth.

Microscopic examination of mixing tank sample 5 showed that yeast cells were present only at a very low level (<0.01 %).

### Conclusions

The results demonstrate that the use of a fibrous (rod-shaped), adsorbent in a dynamic bed system according to the invention is highly efficient for separation of proteins from a simulated fermentation broth comprising 10 vol/vol % of live biomass (baker's yeast). The use of a 40 micron filter unit enables free passage of the yeast cells without clogging and the use of a fibrous adsorbent having a small diameter, of about the same size as the pores in the filter unit, enables a very fast and efficient binding of the target protein even in the presence of the yeast cells.

The same results could not have been achieved with a spherical adsorbent of the same diameter since they would pass through the filter unit and thus not perform a separation in a dynamic system. An attempt to carry out the separation using a filter unit of lower porosity (such as a 10 micron filter) would lead to clogging of the filter since the yeast cells would not be able to pass freely through the filter).

Furthermore, any attempt to perform the separation with a spherical adsorbent of the same diameter in a static (packed bed) system would fail due to clogging, increased back pressure and resulting very low flow rates. On the other hand, the use of an adsorbent with a larger diameter (such as 200 microns) and thus less prone to clogging in a packed bed column would lead to much slower binding of the target protein due to the lower surface area of the adsorbent, and thus a much lower productivity of the separation step.

The results indicate that by choosing the pore size of the filter unit and the diameter of the fibrous adsorbent according to the size of the microorganism in a dynamic bed system it is possible to enable the passing of the microorganism through the filter unit, while the adsorbent, having optimal mass transfer kinetics, is retained by said filter unit.

This feature enables the use of the fibrous adsorbent particles according to the invention for extractive fermentation processes as disclosed herein.

## Claims

1. A method for isolating one or more target biological molecule(s) from a biological composition comprising the steps of
a) providing a separation medium in the form of porous rod-shaped particles that have been derivatised with functional ligands to interact with target biological molecule(s) in a mobile liquid phase, and wherein the porous rod-shaped particles have an average length from 50 µm to 10,000 µm and average diameter from 1µm to 350µm;
b) mixing the separation medium with a biological composition comprising one or more target biological molecule(s), and whereby the one or more target biological molecule is adsorbed onto the separation medium;
c) filtrating the mixture of separation medium and biological composition using a filter to form a dynamic packed bed of the separation medium on the filter, wherein the nominal pore size of the filter is at least 1.5 µm, and wherein the filter is a tangential flow filter;
d) optionally, eluting the target biological molecule from the separation medium while the separation medium is being retained by said filter.

2. The method according to claim 1, wherein the separation medium with the adsorbed target biological molecules is forming a dynamic packed bed on the filter in a flow perpendicular to the tangential flow in the direction of the filtrate flow.

3. The method according to any of the claims 1 or 2, wherein the separation medium after step c) and before step d) is rinsed to remove unbound material.

4. The method according to any of the claims 1 to 3, wherein the pore size of the filter is equal to or larger than the diameter of the rod-shaped particles.

5. The method according to any of the claims 1 to 4, wherein the method comprises a separation apparatus for mixing the separation medium with the biological composition comprising the one or more target biological molecule(s) in step b), and wherein the separation apparatus is capable of circulating the mixture of the biological composition and the separation medium through a tangential flow filtration unit,
and wherein step c) comprises filtrating the mixture of biological composition and separation medium to obtain a retentate comprising the separation medium with adsorbed target biological molecule(s), and a permeate, and recirculating at least part of the retentate on the tangential flow filtration unit, gradually forming a dynamic layer of said separation medium with adsorbed target biological molecule(s), on the retentate site of the filter during the recirculation of the retentate,
and step d) comprises adding an elution liquid to release the target biological molecule from the separation medium, and filtrating the retentate using the tangential flow filtration unit to obtain an eluted retentate comprising the separation medium and a target-containing permeate comprising the target biological molecule, and
optionally, collecting the target-containing permeate and/or the permeate as separated product composition(s).

6. The method according to any of the claims 1 to 5, wherein the separation medium is in the form of porous rod-shaped particles having an average length from 75 µm to 2,000 µm and an average diameter from 5 µm to 180 µm.

7. The method according to any of the clams 1 to 6, wherein the separation medium is in the form of porous rod-shaped particles having an average length from 200 µm to 600 µm and an average diameter from 7 µm to 50 µm.

8. The method according to any of the claims to 7, wherein the porous rod-shaped particles are made from one of the following materials: agarose, dextran, carrageenan, cellulose, regenerated cellulose such as viscose, rayon or lyocell, derivatised celluloses, polyacrylamide, polymethacrylate, polyacrylonitrile, polyethylene oxide, polystyrene, polyamide, polyester, polysulfone, polytetrafluoroethylene, polyvinylidene fluoride, polyvinyl alcohol, silica, alumina, hydroxyapatite or a ceramic material.

9. The method according to any of the claims 1 to 8, wherein the porous rod-shaped particles are made from cellulose, regenerated cellulose, rayon, viscose or lyocell.

10. The method according to any of the claims 1 to 9, wherein the porous rod-shaped particles are derivatised with ionic groups to enable separations based on ion exchange adsorption.

11. The method according to any of the claims 1 to 10, wherein the porous rod-shaped particles are derivatised with groups suitable for Hydrophobic Interaction Chromatography.

12. The method according to any of the claims 1 to 10, wherein the porous rod-shaped particles are derivatised with functional ligands for separations based on affinity interactions.

13. The method according to any of the claims 1 to 11, wherein the biological composition is a fermentation broth.

14. The method according to any of the claims 1 to 12, wherein the biological composition comprising suspended and/or colloid particles.

15. An apparatus for isolating one or more target biological molecule(s) from a biological composition, the apparatus comprising:
- a first inlet 1 to a mixing tank 2 comprising a separation medium in the form of porous rod-shaped particles that have been derivatised with functional ligands to interact with target biological molecule(s) in a mobile liquid phase, and wherein the porous rod-shaped particles have an average length from 50 µm to 10,000 µm and average diameter from 1µm to 350µm;
- said mixing tank 2 having one or more first fluid connections 3 leading fluid from the first inlet 1 to a first end 4 of a filtration unit 5 comprising a filter 6 with a pore size being at least 1,5 µm and wherein the filter is a tangential flow filter;
- and wherein a second end 7 of the filtration unit 5 has a second fluid connection 8 leading retentate back to the mixing tank 2 and the first end of the filtration unit 5;
- the filtration unit 5 having a third fluid connection 9 leading permeate to a first outlet 10.

16. The method according to claim 15, wherein the pore size of the filter is equal to or larger than the diameter of the rod-shaped particles.

## Patentansprüche

1. Verfahren zum Isolieren eines oder mehrerer biologischer Zielmoleküle aus einer biologischen Zusammensetzung, umfassend die folgenden Schritte:
a) Bereitstellen eines Trennmediums in Form poröser stäbchenförmiger Partikel, die mit funktionellen Liganden derivatisiert worden sind, um in einer mobilen flüssigen Phase mit biologischem/biologischen Zielmolekül(en) zu wechselwirken, und wobei die porösen stäbchenförmigen Partikel eine durchschnittliche Länge von 50 µm bis 10.000 µm und einen durchschnittlichen Durchmesser von 1 µm bis 350 µm aufweisen;
b) Mischen des Trennmediums mit einer biologischen Zusammensetzung, umfassend ein oder mehrere biologische(s) Zielmolekül(e), und wodurch das eine oder die mehreren biologische(n) Zielmolekül(e) an dem Trennmedium adsorbiert wird/werden;
c) Filtrieren des Gemisches aus Trennmedium und biologischer Zusammensetzung unter Verwendung eines Filters, um ein dynamisches gepacktes Bett des Trennmediums auf dem Filter zu bilden, wobei die nominelle Porengröße des Filters mindestens 1,5 µm beträgt und wobei der Filter ein Tangentialflussfilter ist;
d) optional Eluieren des biologischen Zielmoleküls von dem Trennmedium, während das Trennmedium von dem genannten Filter zurückgehalten wird.

2. Verfahren nach Anspruch 1, wobei das Trennmedium mit den adsorbierten biologischen Zielmolekülen ein dynamisches gepacktes Bett auf dem Filter in einer Strömung senkrecht zu der Tangentialströmung in Richtung der Filtratströmung bildet.

3. Verfahren nach einem der Ansprüche 1 oder 2, wobei das Trennmedium nach Schritt c) und vor Schritt d) gespült wird, um ungebundenes Material zu entfernen.

4. Verfahren nach einem der Ansprüche 1 bis 3, wobei die Porengröße des Filters gleich oder größer als der Durchmesser der stäbchenförmigen Partikel ist.

5. Verfahren nach einem der Ansprüche 1 bis 4, wobei das Verfahren eine Trenneinrichtung zum Mischen des Trennmediums mit der biologischen Zusammensetzung, umfassend das eine oder die mehreren biologische(n) Zielmolekül(e), in Schritt b) umfasst, und wobei die Trenneinrichtung in der Lage ist, das Gemisch aus der biologischen Zusammensetzung und dem Trennmedium durch eine Tangentialflussfiltrationseinheit zu zirkulieren,
und wobei Schritt c) das Filtrieren des Gemisches aus biologischer Zusammensetzung und Trennmedium, um ein Retentat, umfassend das Trennmedium mit adsorbiertem/adsorbierten biologischem/biologischen Zielmolekül(en), und ein Permeat zu erhalten, und das Rezirkulieren mindestens eines Teils des Retentats auf der Tangentialflussfiltrationseinheit umfasst, wobei sich während der Rezirkulation des Retentats allmählich eine dynamische Schicht des genannten Trennmediums mit adsorbiertem/adsorbierten biologischem/biologischen Zielmolekül(en) auf der Retentatseite des Filters bildet,
und Schritt d) das Zugeben einer Elutionsflüssigkeit, um das biologische Zielmolekül von dem Trennmedium freizusetzen, und das Filtrieren des Retentats unter Verwendung der Tangentialflussfiltrationseinheit umfasst, um ein eluiertes Retentat, umfassend das Trennmedium, und ein zielhaltiges Permeat, umfassend das biologische Zielmolekül, zu erhalten, und
optional das Sammeln des zielhaltigen Permeats und/oder des Permeats als getrennte Produktzusammensetzung(en).

6. Verfahren nach einem der Ansprüche 1 bis 5, wobei das Trennmedium in Form poröser stäbchenförmiger Partikel mit einer durchschnittlichen Länge von 75 µm bis 2.000 µm und einem durchschnittlichen Durchmesser von 5 µm bis 180 µm vorliegt.

7. Verfahren nach einem der Ansprüche 1 bis 6, wobei das Trennmedium in Form poröser stäbchenförmiger Partikel mit einer durchschnittlichen Länge von 200 µm bis 600 µm und einem durchschnittlichen Durchmesser von 7 µm bis 50 µm vorliegt.

8. Verfahren nach einem der Ansprüche bis 7, wobei die porösen stäbchenförmigen Partikel aus einem der folgenden Materialien hergestellt sind: Agarose, Dextran, Carrageenan, Cellulose, regenerierte Cellulose wie Viskose, Rayon oder Lyocell, derivatisierte Cellulosen, Polyacrylamid, Polymethacrylat, Polyacrylnitril, Polyethylenoxid, Polystyrol, Polyamid, Polyester, Polysulfon, Polytetrafluorethylen, Polyvinylidenfluorid, Polyvinylalkohol, Siliciumdioxid, Aluminiumoxid, Hydroxylapatit oder ein keramisches Material.

9. Verfahren nach einem der Ansprüche 1 bis 8, wobei die porösen stäbchenförmigen Partikel aus Cellulose, regenerierter Cellulose, Rayon, Viskose oder Lyocell hergestellt sind.

10. Verfahren nach einem der Ansprüche 1 bis 9, wobei die porösen stäbchenförmigen Partikel mit ionischen Gruppen derivatisiert sind, um Trennungen auf Basis von Ionenaustauschadsorption zu ermöglichen.

11. Verfahren nach einem der Ansprüche 1 bis 10, wobei die porösen stäbchenförmigen Partikel mit Gruppen derivatisiert sind, die für die hydrophobe Interaktionschromatographie geeignet sind.

12. Verfahren nach einem der Ansprüche 1 bis 10, wobei die porösen stäbchenförmigen Partikel mit funktionellen Liganden für Trennungen auf Basis von Affinitätswechselwirkungen derivatisiert sind.

13. Verfahren nach einem der Ansprüche 1 bis 11, wobei die biologische Zusammensetzung eine Fermentationsbrühe ist.

14. Verfahren nach einem der Ansprüche 1 bis 12, wobei die biologische Zusammensetzung suspendierte und/oder kolloidale Partikel umfasst.

15. Einrichtung zum Isolieren eines oder mehrerer biologischer Zielmoleküle aus einer biologischen Zusammensetzung, wobei die Einrichtung Folgendes umfasst:
- einen ersten Einlass 1 zu einem Mischtank 2, umfassend ein Trennmedium in Form poröser stäbchenförmiger Partikel, die mit funktionellen Liganden derivatisiert worden sind, um in einer mobilen flüssigen Phase mit biologischem/biologischen Zielmolekül(en) zu wechselwirken, und wobei die porösen stäbchenförmigen Partikel eine durchschnittliche Länge von 50 µm bis 10.000 µm und einen durchschnittlichen Durchmesser von 1µm bis 350µm aufweisen;
- wobei der genannte Mischtank 2 eine oder mehrere erste Fluidverbindungen 3 aufweist, die Fluid von dem ersten Einlass 1 zu einem ersten Ende 4 einer Filtrationseinheit 5 leiten, umfassend einen Filter 6 mit einer Porengröße von mindestens 1,5 µm, und wobei der Filter ein Tangentialflussfilter ist;
- und wobei ein zweites Ende 7 der Filtrationseinheit 5 eine zweite Fluidverbindung 8 aufweist, die Retentat zurück zu dem Mischtank 2 und dem ersten Ende der Filtrationseinheit 5 leitet;
- wobei die Filtrationseinheit 5 eine dritte Fluidverbindung 9 aufweist, die Permeat zu einem ersten Auslass 10 leitet.

16. Verfahren nach Anspruch 15, wobei die Porengröße des Filters gleich oder größer als der Durchmesser der stäbchenförmigen Partikel ist.

## Revendications

1. Procédé d'isolement d'une ou de plusieurs molécule(s) biologique(s) cible(s) à partir d'une composition biologique, comprenant les étapes consistant à :
a) fournir un milieu de séparation sous la forme de particules poreuses en forme de bâtonnets qui ont été transformées en dérivés avec des ligands fonctionnels afin d'interagir avec une/des molécule(s) biologique(s) cible(s) dans une phase liquide mobile, et les particules poreuses en forme de bâtonnets ayant une longueur moyenne de 50 µm à 10 000 µm et un diamètre moyen de 1 µm à 350 µm ;
b) mélanger le milieu de séparation avec une composition biologique comprenant une ou plusieurs molécule(s) biologique(s) cible(s), et ce par quoi lesdites une ou plusieurs molécule(s) biologique(s) cible(s) est/sont adsorbée(s) sur le milieu de séparation ;
c) filtrer le mélange de milieu de séparation et composition biologique à l'aide d'un filtre pour obtenir un lit compact dynamique constitué du milieu de séparation sur le filtre, la taille nominale de pore du filtre étant d'au moins 1,5 µm, et le filtre étant un filtre à flux tangentiel ;
d) en option, éluer la molécule biologique cible à partir du milieu de séparation tandis que le milieu de séparation est retenu par ledit filtre.

2. Procédé selon la revendication 1, dans lequel le milieu de séparation comportant les molécules biologiques cibles adsorbées forme un lit compact dynamique sur le filtre dans un flux perpendiculaire au flux tangentiel dans la direction de l'écoulement du filtrat.

3. Procédé selon l'une quelconque des revendications 1 et 2, dans lequel le milieu de séparation est rincé après l'étape c) et avant l'étape d) afin d'éliminer les matières non liées.

4. Procédé selon l'une quelconque des revendications 1 à 3, dans lequel la taille de pore du filtre est égale ou supérieure au diamètre des particules en forme de bâtonnets.

5. Procédé selon l'une quelconque des revendications 1 à 4, dans lequel le procédé comprend un appareil de séparation destiné à mélanger le milieu de séparation avec la composition biologique comprenant lesdites une ou plusieurs molécule(s) biologique(s) cible(s) dans l'étape b), et dans lequel l'appareil de séparation est capable de faire circuler le mélange de la composition biologique et du milieu de séparation à travers une unité de filtration à flux tangentiel,
et dans lequel l'étape c) comprend la filtration du mélange de composition biologique et milieu de séparation pour obtenir un rétentat comprenant le milieu de séparation comportant une/des molécule(s) biologique(s) cible(s) adsorbées, et un perméat, et la recirculation d'au moins une partie du rétentat sur l'unité de filtration à flux tangentiel, formant progressivement une couche dynamique dudit milieu de séparation comportant une/des molécule(s) biologique(s) cible(s) adsorbée(s), du côté rétentat du filtre pendant la recirculation du rétentat,
et l'étape d) comprend l'addition d'un liquide d'élution pour libérer la molécule biologique cible d'avec le milieu de séparation, et la filtration du rétentat à l'aide de l'unité de filtration à flux tangentiel pour obtenir un rétentat élué comprenant le milieu de séparation et un perméat contenant la cible, comprenant la molécule biologique cible, et
en option, la collecte du perméat contenant la cible et/ou du perméat en tant que composition(s) de produit séparée(s).

6. Procédé selon l'une quelconque des revendications 1 à 5, dans lequel le milieu de séparation est sous la forme de particules poreuses en forme de bâtonnets ayant une longueur moyenne de 75 µm à 2 000 µm et un diamètre moyen de 5 µm à 180 µm.

7. Procédé selon l'une quelconque des revendications 1 à 6, dans lequel le milieu de séparation est sous la forme de particules poreuses en forme de bâtonnets ayant une longueur moyenne de 200 µm à 600 µm et un diamètre moyen de 7 µm à 50 µm.

8. Procédé selon l'une quelconque des revendications à 7, dans lequel les particules poreuses en forme de bâtonnets sont constituées de l'un des matériaux suivants : agarose, dextrane, carraghénane, cellulose, cellulose régénérée telle que viscose, rayonne ou lyocell, celluloses transformées en dérivés, polyacrylamide, polyméthacrylate, polyacrylonitrile, polyoxyéthylène, polystyrène, polyamide, polyester, polysulfone, polytétrafluoroéthylène, poly(fluorure de vinylidène), poly(alcool vinylique), silice, alumine, hydroxyapatite ou un matériau céramique.

9. Procédé selon l'une quelconque des revendications 1 à 8, dans lequel les particules poreuses en forme de bâtonnets sont constituées de cellulose, cellulose régénérée, rayonne, viscose ou lyocell.

10. Procédé selon l'une quelconque des revendications 1 à 9, dans lequel les particules poreuses en forme de bâtonnets sont transformées en dérivés avec des groupes ioniques pour permettre des séparations basées sur l'adsorption par échange d'ions.

11. Procédé selon l'une quelconque des revendications 1 à 10, dans lequel les particules poreuses en forme de bâtonnets sont transformées en dérivé avec des groupes appropriés à la chromatographie par interaction hydrophobe.

12. Procédé selon l'une quelconque des revendications 1 à 10, dans lequel les particules poreuses en forme de bâtonnets sont transformées en dérivés avec des ligands fonctionnels pour des séparations fondées sur des interactions d'affinité.

13. Procédé selon l'une quelconque des revendications 1 à 11, dans lequel la composition biologique est un bouillon de fermentation.

14. Procédé selon l'une quelconque des revendications 1 à 12, dans lequel la composition biologique comprend des particules en suspension et/ou colloïdales.

15. Appareil destiné à l'isolement d'une ou de plusieurs molécule(s) biologique(s) cible(s) à partir d'une composition biologique, l'appareil comprenant :
- une première entrée (1) vers une cuve de mélange (2) comprenant un milieu de séparation sous la forme de particules poreuses en forme de bâtonnets qui ont été transformées en dérivés avec des ligands fonctionnels pour interagir avec une/des molécule(s) biologique(s) cible(s) dans une phase liquide mobile, et les particules poreuses en forme de bâtonnets ayant une longueur moyenne de 50 µm à 10 000 µm et un diamètre moyen de 1 µm à 350 µm ;
- ladite cuve de mélange (2) comportant un ou plusieurs premiers conduits de fluide (3) acheminant du fluide depuis la première entrée (1) vers une première extrémité (4) d'une unité de filtration (5) comprenant un filtre (6) ayant une taille de pore d'au moins 1,5 µm, et le filtre étant un filtre à flux tangentiel ;
- et une deuxième extrémité (7) de l'unité de filtration (5) comportant un deuxième conduit de fluide (8) renvoyant le rétentat vers la cuve de mélange (2) et la première extrémité de l'unité de filtration (5) ;
- l'unité de filtration (5) comportant un troisième conduit de fluide (9) acheminant le perméat vers une première sortie (10).

16. Procédé selon la revendication 15, dans lequel la taille de pore du filtre est égale ou supérieure au diamètre des particules en forme de bâtonnets.
